Europäisches Patentamt

European Patent Office

Office européen des brevets

(19)

(11) Veröffentlichungsnummer: **0 208 653**
**B1**

(12) **EUROPÄISCHE PATENTSCHRIFT**

(45) Veröffentlichungstag der Patentschrift:
30.08.89

(51) Int. Cl.⁴: **A 61 F 5/47**

(21) Anmeldenummer: 86810275.7

(22) Anmeldetag: 16.06.86

(54) **Okklusivpessar.**

(30) Priorität: 11.07.85 CH 3015/85

(43) Veröffentlichungstag der Anmeldung:
14.01.87 Patentblatt 87/03

(45) Bekanntmachung des Hinweises auf die Patenterteilung: 30.08.89 Patentblatt 89/35

(84) Benannte Vertragsstaaten:
AT BE CH DE FR GB IT LI LU NL SE

(56) Entgegenhaltungen:
EP–A– 0 089 035
EP–A– 0 114 440
EP–A– 0 153 021
CH–A– 505 617
DE–A– 2 803 685
FR–A– 2 085 578
US–A– 3 405 711

(73) Patentinhaber: Cimber, Hugo, Dr. med.
Neufeldstrasse 134
CH-3012 Bern (CH)

(72) Erfinder: Cimber, Hugo, Dr. med.
Neufeldstrasse 134
CH-3012 Bern (CH)

(74) Vertreter: Bovard, Fritz Albert et al
Bovard AG Patentanwälte VSP Optingenstrasse 16
CH-3000 Bern 25 (CH)

# Beschreibung

Gegenstand der vorliegenden Erfindung ist ein Okklusivpessar gemäss dem Oberbegriff des einzigen Patentanspruches.

Es sind Okklusivpessare bekannt, bei welchen zwei voneinander weg aufspreizbare Aeste aus einem Röhrchen ausgestossen werden, wobei die Aeste an ihren Enden Verschlussorgane tragen. So ist beispielsweise aus der FR-A-2 085 578 ein Okklusivpessar bekannt, der mittels eines Röhrchens in die Gebärmutter eingeführt wird, wobei ein Tragbalken mit zwei an dessen in der Einführrichtung vorderem Ende angelenkten Aesten vorgesehen ist, derart, dass sich die Aeste nach ihrem Ausstossen aus dem Röhrchen seitlich voneinander wegspreizen. An ihren freien Enden tragen die Aeste je ein kugeliges Abschlussorgan, welche Abschlussorgane dazu bestimmt und geeignet sind, die Mündungen der Eileiter in die Gebärmutter zu verschliessen. Die Aeste sind von ungefähr gleicher Länge, so dass die beiden Abschlussorgane beim Einziehen des Pessars in das Röhrchen nebeneinander zu liegen kommen, und das Röhrchen somit einen vergleichsweise grossen Durchmesser aufweisen muss.

Aehnlich liegen die Verhältnisse in bezug auf den Inhalt der EP-A-114 440, indem auch bei dem dort beschriebenen Pessar die beiden Abschlussstücke beim Einziehen des Pessars in das Röhrchen nebeneinander zu liegen kommen.

Das Bestreben bei Okklusivpessaren geht nun dahin, den Durchmesser des Röhrchens möglichst klein zu halten, da bekanntlich die Einführung solcher Röhrchen umso leichter und angenehmer wird, je kleiner der Röhrchendurchmesser ist. Dieser Durchmesser wieder hängt im wesentlichen davon ab, in welcher gegenseitigen Stellung die am Ende dieser Aeste befestigten Abschlussorgane in das Röhrchen eingezogen werden können. Die gegenseitige Stellung, welche sich in hervorragender Weise dazu eignet, das genannte Ziel zu erreichen, ist nun eine solche, bei der die kugelförmigen Abschlussorgane bei in das Röhrchen eingezogenen Aesten, in der Einzugsrichtung gesehen, hintereinander und nicht nebeneinander zu liegen kommen.

Dabei muss weiter die zusätzliche Bedingung erfüllt werden, dass nämlich die Länge der beiden Aeste, gemessen von ihrem Anlenkpunkt am Tragbalken bis zum freien Ende der Abschlussorgane, mindestens angenähert die gleiche ist.

Dem erfindungsgemässen Okklusivpessar lag demzufolge die Aufgabe zugrunde, einen der Aeste so auszubilden, dass wenn diese Aeste in das Röhrchen zurückgezogen werden, die Abschlussorgane zwangsläufig in eine solche gegenseitige Lage zueinander gelangen, dass die Verwendung eines möglichst dünnen Röhrchens ermöglicht wird. Die Schwierigkeit bei solchen Okklusivpessaren besteht nun darin, dass beim Einzug der Aeste in das Röhrchen diese Aeste sich an der richtigen Stelle biegen, so dass sie gemeinsam in einer vorbestimmten Stellung, und zwar hintereinander und nicht nebeneinander, in das Röhrchen eingezogen werden können.

Dem erfindungsgemässen Okklusivpessar lag die Aufgabe zugrunde, eine solche Verbiegung mindestens des einen Astes auf einfache Weise zu gewährleisten.

Dies gelingt durch die Anordnung der im kennzeichnenden Teil des Patentanspruches erwähnten Merkmale.

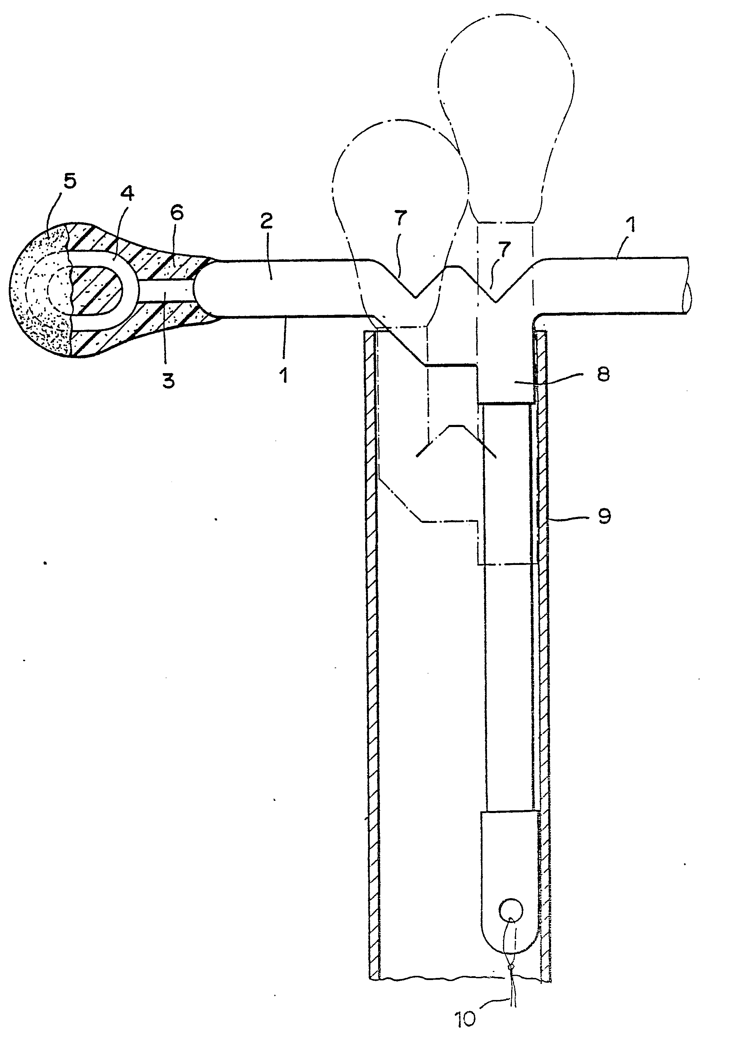

In der Zeichnung ist eine beispielsweise Ausführungsform des Erfindungsgegenstandes dargestellt, und zwar zeigt die einzige Figur in vergrössertem Massstab einen teilweisen Schnitt eines der in ausgestossenem Zustand seitlich abstehenden Aeste.

Beim dargestellten Ausführungsbeispiel sind die Aeste 1 zweistückig ausgeführt und weisen je ein Positionierungsstück 2 auf, an welches ein dünner, biegsamer und als solcher beweglicher Fortsatz 3 anschliesst, der das betreffende Positionierungsstück 2 mit einem durchbrochenen, ösenförmigen Tragstück 4 verbindet. Dieses Tragstück 4, welches der Verbesserung der Positionierungsmöglichkeit unter Zuhilfenahme von Unterschallwellen dient, könnte an sich jede eine Verankerung begünstigende Formgebung aufweisen, vorausgesetzt, dass es aus Gründen der Sichtbarmachung durch Ultraschallwellen mindestens eine Durchbrechung besitzt. Beim dargestellten Ausführungsbeispiel ist das Tragstück 4 ringförmig ausgebildet. Auf dieses Tragstück 4 aufgebracht ist die Aussenhaut eines birnenförmigen Abschlussorganes 5, dessen Befestigungsteil 6 den Fortsatz 3 umgibt und auf das Ende des Positionierungsstückes 2 aufgebracht ist, welches letzte aus beliebig steifem Material, vorzugsweise einem geeigneten Kunststoff, besteht und auf seiner Oberseite zwei Einkerbungen 7 aufweist, durch welche eine Querschnittschwächung an der betreffenden Stelle hervorgerufen wird. Dabei ist die Formgebung so gewählt, dass die birnenförmigen Abschlussorgane 5 zusammen mit den jeweiligen Befestigungsteilen 6 einen birnenförmigen Körper bilden. Das steife Positionierstück presst das weiche Abschlussorgan gegen die Wand der Gebärmutter und bewirkt somit eine Fixierung des gesamten Okklusivpessars in der Gebärmutter, ohne dass die Gefahr einer Perforation der Gebärmutterwand besteht. Die Steife des Positionierstückes kann bis zum Maximum erhöht werden, was eine spontane Ausstossung des Okklusivpessars verhindern kann.

Wenn also, wie dies bei Okklusivpessaren der betreffenden Art üblich ist, der vorzugsweise mit einem nicht dargestellten Kupferdraht umwickelte Trägerbalken 8 samt seinen Aesten 1 aus dem Einführrohr 9 ausgestossen wird, so spreizen sich die beiden Aeste 1 in entgegengesetzter Richtung voneinander weg. Dabei sind die Abmessungen so gewählt, dass die ausgestossenen Aeste 1 mindestens ungefähr in der Richtung auf die Mündungen der Eileiter in der Gebärmutter gerichtet sind. Die weichen und mit den Positionierungsstücken 2 flexibel verbundenen Abschlussorgane 5 gelangen nun dank ihrer Form genau an die abzuschliessende Stelle, und dies auch dann, wenn die Richtung der Positionierungsstücke 2 nicht ganz genau sein sollte.

Selbst wenn also die Positionierungsstücke 2 nicht unbedingt auf die Mündungen der Eileiter in der Gebärmutter weisen sollten, so ist durch die beschriebene Anordnung und insbesondere die flexible Verbindung zwischen den Positionierungsstücken 2 und den birnenförmigen Abschlussorganen 5 der Abschluss der Mündungen der Eileiter in der Gebärmutter sichergestellt.

Zum Zwecke der Entfernung des Pessars aus seiner Wirkstellung genügt es, den Tragbalken 8 vermittels des Rückzugorganes 10 nach unten zu ziehen, so dass die beiden Aeste 1 sich wieder gegeneinander zu bewegen. Dank des Umstandes, dass der dargestellte Ast 1 sich bereits an der Stelle der durch die äusseren Einkerbungen 7 gebildeten Querschnittschwächung biegt, wogegen die Biegung des anderen Astes 1 erst im Bereiche des Tragbalkens 8 erfolgt, kommen die beiden birnenförmigen Abschlusskörper 5 am Ende der beiden Aeste 1 untereinander zu liegen und finden so innerhalb der lichten Weite des Einführrohres 9 Platz, was durch die Birnenform der Abschlussorgane insofern erleichtert wird, als die Lage der Einkerbungen 7 zur Folge hat, dass, wenn die Aeste sich in der nicht aufgespreizten Lage befinden, das dickere Ende des einen Abschlussorganes gegen den dünneren Hals des anderen Abschlussorganes anliegt.

## Patentanspruch

Mittels eines Röhrchens in die Gebärmutter einzuführender Okklusivpessar mit einem Tragbalken und zwei an dessen in der Einführrichtung vorderem Ende derart angelenkten Aesten, dass sich diese nach ihrem Ausstossen aus dem Röhrchen seitlich voneinander wegspreizen, wobei die Aeste an ihren freien Enden je ein kugeliges Abschlussorgan tragen, welche Abschlussorgane dazu bestimmt und geeignet sind, die Mündungen der Eileiter in die Gebärmutter zu verschliessen, wobei die Aeste je aus einem vergleichsweise steifen . Positionierungsstück sowie einem weichen Abschlussorgan bestehen, wobei die Abschlussorgane auf einem dünnen, biegsamen und beweglichen Fortsatz am freien Ende jedes Astes angebracht sind, welche Fortsätze nicht nur der Verbindung der Positionierungsstücke mit einem Abschlussorgan, sondern gleichzeit noch der Halterung eines durchbrochenen Lokalisierungsorganes dienen, dadurch gekennzeichnet, dass einer der beiden Aeste an seinem Anlenkende am Tragbalken eine Querschnittschwächung aufweist, und die Querschnittschwächung aus mindestens einer Einkerbung auf der Oberseite des betreffenden Astes besteht.

## Claim

Occlusive pessary to be introduced into the uterus by means of a small tube, having a supporting beam and two branches hinged to the front end thereof, in the direction of introduction, in such a way that these branches spread away from each other laterally after their expulsion from the small tube, the branches each bearing at their free ends a spherical closure part, which closure parts are intended and suitable for occluding the mouths of the Fallopian tubes into the uterus, the branches each consisting of a comparatively stiff positioning piece as well as a soft closure part, the closure parts being affixed to a thin, flexible and movable extension at the free end of each branch, which extensions serve not only for the connection of the positioning pieces to a closure part but simultaneously also for the mounting of a perforated localization part, characterized in that one of the two branches has a cross-sectional weakening at its end hinged to the support beam, and the cross-sectional weakening consists of at least one notch on the upper side of the respective branch.

## Revendication

Un pessaire occlusif pouvant être introduit dans la matrice au moyen d'un petit tube, avec un support et deux branches articulées à l'extrémité avant dans la direction d'introduction, qui s'écartent l'une de l'autre de chaque côté après avoir été poussées hors du petit tube, avec les branches portant chacune à leurs extrémités libres un organe terminal sphérique, lesquels organes terminaux sont destinés et appropriés à fermer les orifices des. trompes de la matrice, les branches possédant chacune une pièce de positionnement comparativement rigide ainsi qu'un organe terminal fléchissant, les organes terminaux étant commencés par un prolongement fin, flexible et mobile, aux extrémités libres de chaque branche, lesquels prolongements ne servent pas seulement de connection des pièces de positionnement avec un organe terminal, mais en même temps servent de fixation à un organe de localisation percé, caractérisé en ce que l'une des deux branches présente une diminution de section à son articulation au support, et la diminution de section comprenant au moins une entaille sur la surface supérieure de la branche concernée.